# EUROPEAN PATENT APPLICATION

(11) **EP 1 955 699 A1**
(43) Date of publication of application: **13.08.2008**
(21) Application number: 07101971.5
(22) Date of filing: 08.02.2007
(51) Int. Cl.: A61K 31/496, A61P 25/20

(54) **Use of flibanserin for the treatment of insomnia**

(71) Applicant: Boehringer Ingelheim Pharma GmbH & Co. KG, 55216 Ingelheim am Rhein (DE)
(72) Inventor: The designation of the inventor has not yet been filed
(74) Representative: Hammann, Heinz

(57) **Abstract**

The invention relates to the use of Flibanserin for the preparation of medicaments useful for the treatment of Insomnia.

## Description

The invention relates to the use of Flibanserin for the preparation of medicaments useful for the treatment of Insomnia.

### Description of the invention

The compound 1-[2-(4-(3-trifluoromethyl-phenyl)piperazin-1-yl)ethyl]-2,3-dihydro-1H-benzimidazol-2-one (flibanserin) is disclosed in form of its hydrochloride in European Patent Application EP-A-526434 and has the following chemical structure:

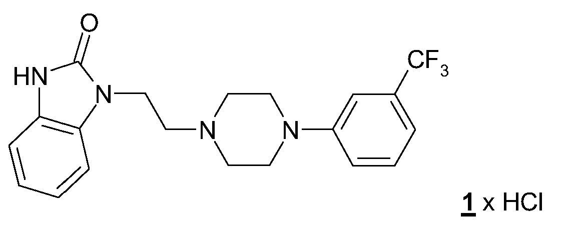

Flibanserin shows affinity for the 5-HT_{1A} and 5-HT₂-receptor. It is therefore a promising therapeutic agent for the treatment of a variety of diseases, for instance depression, schizophrenia, and anxiety.

The instant invention relates to the use of flibanserin, optionally in form the free base, the pharmacologically acceptable acid addition salts and/or optionally in form of the hydrates and/or solvates thereof for the preparation of medicaments useful for the treatment and/or prevention of Insomnia.

Another embodiment of the invention relates to a method for the treatment and/or prevention of Insomnia comprising the administration of a therapeutically effective amount of flibanserin, optionally in form the free base, the pharmacologically acceptable acid addition salts and/or optionally in form of the hydrates and/or solvates thereof.

Sleep disorders are organized into sections according to presumed etiology like Dyssomnias, which are characterized by abnormalities in the amount, quality, or timing of sleep and Parasomnias which are charcterized by abnormal behavioural or physiological events occurring in association with sleep, specific sleep stages, or sleep-wake transitions.

Dyssomnias are subdivided into Insomnia, Hypersomnia, Narcolepsy, Breathing Related Sleep Disorder, Circadian Rhythm Sleep Disorder and Dyssomnia not otherwise specified.
The Parasomnias are disorders characterized by abnormal behavioural or physiological events occurring in association with sleep, specific sleep stages , or sleep-wake transitions. Unlike dyssomnias, parasomnias do not involve abnormalities of the mechanisms generating sleep-wake states, nor of the timing of sleep and wakefulness. Parasomnias are subdivided into Nightmare Disorder, Sleep Terror Disorder, Sleepwalking Disorder and Parasomnia not otherwise specified (Diagnostic and Statistical Manual of Mental Disorders, 4th edition, Text Revision. Washington DC, American Psychiatric Association, 2000).

Within the present invention, the term "Insomnia " is intended to mean a Dyssomnia characterized by difficulties initiating or maintaining sleep and/or characterized by nonrestorative sleep, for at least one month and which causes clinically significant distress or impairment in social, occupational, or other important areas of functioning. The term includes primary Insomnia and Insomnia secondary to another mental disorder, a general medical condition, or induced by a substance (hereafter referred to as secondary Insomnia). Examples of secondary insomnia include but are not limited to insomnia accompanying a circadian rhythm sleep disorder, or Insomnia due to occasional stress.
Substance-induced Insomnia often occurs during intoxication with one or more of the following classes of substances: alcohol, amphetamine (incl. related substances), anxiolytics, caffeine, cocaine, opioids, sedatives, and hypnotics.

Accordingly, the instant invention further relates to the use of flibanserin, optionally in form the free base, the pharmacologically acceptable acid addition salts and/or optionally in form of the hydrates and/or solvates thereof for the preparation of medicaments for the treatment and/or prevention of primary Insomnia, Insomnia secondary to another mental disorder, Insomnia due to a general medical condition, and Insomnia induced by a substance (for instance, induced by alcohol, amphetamines, anxiolytics, caffeine, cocaine, opioids, sedatives, and hypnotics).

In addition the present invention relates to a method for the treatment and/or prevention of any one of the above mentioned diseases and conditions, comprising the administration of a therapeutically effective amount of flibanserin, optionally in form the free base, the pharmacologically acceptable acid addition salts and/or optionally in form of the hydrates and/or solvates thereof.

Flibanserin can optionally used in form of the free base, in form of its pharmaceutically acceptable acid addition salts and/or optionally in form of the hydrates and/or solvates thereof. Suitable acid addition salts include for example those of the acids selected from, succinic acid, hydrobromic acid, acetic acid, fumaric acid, maleic acid, methanesulphonic acid, lactic acid, phosphoric acid, hydrochloric acid, sulphuric acid, tartaric acid and citric acid. Mixtures of the abovementioned acid addition salts may also be used. From the aforementioned acid addition salts the hydrochloride and the hydrobromide, particularily the hydrochloride, are preferred. If flibanserin is used in form of the free base, it is preferably used in form of flibanserin polymorph A as disclosed in WO 03/014079.

Flibanserin, optionally used in form of its pharmaceutically acceptable acid addition salts, may be incorporated into the conventional pharmaceutical preparation in solid, liquid or spray form. The composition may, for example, be presented in a form suitable for oral, rectal, parenteral administration or for nasal inhalation: preferred forms includes for example, capsules, tablets, coated tablets, ampoules, suppositories and nasal spray.

The active ingredient may be incorporated in excipients or carriers conventionally used in pharmaceutical compositions such as, for example, talc, arabic gum, lactose, gelatine, magnesium stearate, corn starch, acqueous or non acqueous vehicles, polyvynil pyrrolidone, semisynthetic glicerides of fatty acids, benzalconium chloride, sodium phosphate , EDTA, polysorbate 80. The compositions are advantageously formulated in dosage units, each dosage unit being adapted to supply a single dose of the active ingredient. The dosis range applicable per day is between 0.1 to 400, preferably between 1.0 to 300, more preferably between 2 to 200 mg.
Each dosage unit may conveniently contain from 0,01 mg to 100 mg, preferably from 0,1 to 50 mg.

The dosage units are administered to the patient 1, 2, 3, or 4 times daily. It is preferred that the compounds of the invention be administered either three or fewer times, more preferably once or twice daily consecutively over a period of time.

Preferably, the dose is administered to a patient in the morning and the evening, more preferably once in the morning (25 to 200 mg of flibanserin) and once in the evening (25 to 200 mg of flibanserin), most preferably once in the evening only (50, 100 or 200 mg of flibanserin) consecutively over a period of time.

Suitable tablets may be obtained, for example, by mixing the active substance(s) with known excipients, for example inert diluents such as calcium carbonate, calcium phosphate or lactose, disintegrants such as corn starch or alginic acid, binders such as starch or gelatine, lubricants such as magnesium stearate or talc and/or agents for delaying release, such as carboxymethyl cellulose, cellulose acetate phthalate, or polyvinyl acetate. The tablets may also comprise several layers.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number or layers to achieve delayed release, possibly using the excipients mentioned above for the tablets.

Syrups or elixirs containing the active substances or combinations thereof according to the invention may additionally contain a sweetener such as saccharine, cyclamate, glycerol or sugar and a flavour enhancer, e.g of. a flavouring such as vanilline or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

Solutions for injection are prepared in the usual way, e.g of. with the addition of preservatives such as p-hydroxybenzoates, or stabilisers such as alkali metal salts of ethylenediamine tetraacetic acid, and transferred into injection vials or ampoules.

Capsules containing one or more active substances or combinations of active substances may for example be prepared by mixing the active substances with inert carriers such as lactose or sorbitol and packing them into gelatine capsules.

Suitable suppositories may be made for example by mixing with carriers provided for this purpose, such as neutral fats or polyethyleneglycol or the derivatives thereof.

The Examples which follow illustrate the present invention without restricting its scope:

### Examples of pharmaceutical formulations

| A) | Tablets | per tablet |
|---|---|---|
| | flibanserin hydrochloride | 100 mg |
| | lactose | 240 mg |
| | corn starch | 340 mg |
| | polyvinylpyrrolidone | 45 mg |
| | magnesium stearate | 15 mg |
| | | 740 mg |

The finely ground active substance, lactose and some of the corn starch are mixed together. The mixture is screened, then moistened with a solution of polyvinylpyrrolidone in water, kneaded, wet-granulated and dried. The granules, the remaining corn starch and the magnesium stearate are screened and mixed together. The mixture is compressed to produce tablets of suitable shape and size.

| B) | Tablets | per tablet |
|---|---|---|
| | flibanserin hydrochloride | 80 mg |
| | corn starch | 190 mg |
| | lactose | 55 mg |
| | microcrystalline cellulose | 35 mg |
| | polyvinylpyrrolidone | 15 mg |
| | sodium-carboxymethyl starch | 23 mg |
| | magnesium stearate | 2 mg |
| | | 400 mg |

The finely ground active substance, some of the corn starch, lactose, microcrystalline cellulose and polyvinylpyrrolidone are mixed together, the mixture is screened and worked with the remaining corn starch and water to form a granulate which is dried and screened. The sodium-carboxymethyl starch and the magnesium stearate are added and mixed in and the mixture is compressed to form tablets of a suitable size.

| C) | Coated tablets | per coated tablet |
|---|---|---|
| | flibanserin hydrochloride | 5 mg |
| | corn starch | 41.5 mg |
| | lactose | 30 mg |
| | polyvinylpyrrolidone | 3 mg |
| | magnesium stearate | 0.5 mg |
| | | 80 mg |

The active substance, corn starch, lactose and polyvinylpyrrolidone are thoroughly mixed and moistened with water. The moist mass is pushed through a screen with a 1 mm mesh size, dried at about 45°C and the granules are then passed through the same screen. After the magnesium stearate has been mixed in, convex tablet cores with a diameter of 6 mm are compressed in a tablet-making machine. The tablet cores thus produced are coated in known manner with a covering consisting essentially of sugar and talc. The finished coated tablets are polished with wax.

| D) | Capsules | per capsule |
|---|---|---|
| | flibanserin hydrochloride | 1 50 mg |
| | Corn starch | 268.5 mg |
| | Magnesium stearate | 1.5 mg |
| | | 420 mg |

The substance and corn starch are mixed and moistened with water. The moist mass is screened and dried. The dry granules are screened and mixed with magnesium stearate. The finished mixture is packed into size 1 hard gelatine capsules.

| E) | Ampoule solution | |
|---|---|---|
| | flibanserin hydrochloride | 50 mg |
| | sodium chloride | 50 mg |
| | water for inj. | 5 ml |

The active substance is dissolved in water at its own pH or optionally at pH 5.5 to 6.5 and sodium chloride is added to make it isotonic. The solution obtained is filtered free from pyrogens and the filtrate is transferred under aseptic conditions into ampoules which are then sterilised and sealed by fusion.

| F) Suppositories | | |
|---|---|---|
| | flibanserin hydrochloride | 50 mg |
| | solid fat | 1650 mg |
| | | 1700 mg |

The hard fat is melted. At 40°C the ground active substance is homogeneously dispersed. It is cooled to 38°C and poured into slightly chilled suppository moulds.

In a particular preferred embodiment of the instsnt invention, flibanserin is administered in form of specific film coated tablets. Examples of these preferred formulations are listed below. The film coated tablets listed below can be manufactured according to procedures known in the art (see hereto WO 03/097058).

### G) Film coated tablet

### Core

| **Constituents** | **mg/tablet** |
|---|---|
| Flibanserin | 25.000 |
| Lactose monohydrate | 71.720 |
| Microcrystalline cellulose | 23.905 |
| HPMC (Methocel E5) | 1.250 |
| Carboxymethylcellulose sodium | 2.500 |
| Magnesium stearate | 0.625 |

### Coating

| **Constituents** | **mg/ tablet** |
|---|---|
| HPMC (Methocel E5) | 1.440 |
| Polyethylene Glycol 6000 | 0.420 |
| Titanium dioxide | 0.600 |
| Talc | 0.514 |
| Iron oxide red | 0.026 |
| | |
| **Total Film coated tablet** | **128.000** |

### H) Film coated tablet

### Core

| **Constituents** | **mg/tablet** |
|---|---|
| Flibanserin | 50.000 |
| Lactose monohydrate | 143.440 |
| Microcrystalline cellulose | 47.810 |
| HPMC (e.g. Pharmacoat 606) | 2.500 |
| Carboxymethylcellulose sodium | 5.000 |
| Magnesium stearate | 1.250 |

### Coating

| **Constituents** | **mg/ tablet** |
|---|---|
| HPMC (e.g. Pharmacoat 606) | 2.400 |
| Polyethylene Glycol 6000 | 0.700 |
| Titanium dioxide | 1.000 |
| Talc | 0.857 |
| Iron oxide red | 0.043 |
| | |
| **Total Film coated tablet** | **255.000** |

### I) Film coated tablet

### Core

| **Constituents** | **mg/tablet** |
|---|---|
| Flibanserin | 100.000 |
| Lactose monohydrate | 171.080 |
| Microcrystalline cellulose | 57.020 |
| HPMC (e.g. Methocel E5) | 3.400 |
| Carboxymethylcellulose sodium | 6.800 |
| Magnesium stearate | 1.700 |

### Coating

| **Constituents** | **mg/ tablet** |
|---|---|
| HPMC (e.g. Methocel E5) | 3.360 |
| Polyethylene Glycol 6000 | 0.980 |
| Titanium dioxide | 1.400 |
| Talc | 1.200 |
| Iron oxide red | 0.060 |
| | |
| **Total Film coated tablet** | **347.000** |

### J) Film coated tablet

### Core

| **Constituents** | **mg/tablet** |
|---|---|
| Flibanserin | 2.000 |
| Dibasic Calciumphosphate, anhydrous | 61.010 |
| Microcrystalline cellulose | 61.010 |
| HPMC (Methocel E5) | 1.950 |
| Carboxymethylcellulose sodium | 2.600 |
| Colloidal silicon dioxide | 0.650 |
| Magnesium stearate | 0.780 |

### Coating

| **Constituents** | **mg/ tablet** |
|---|---|
| HPMC (Methocel E5) | 1.440 |
| Polyethylene Glycol 6000 | 0.420 |
| Titanium dioxide | 0.600 |
| Talc | 0.514 |
| Iron oxide red | 0.026 |
| **Total Film coated tablet** | **133.000** |

### K) Film coated tablet

### Core

| **Constituents** | **mg/tablet** |
|---|---|
| Flibanserin | 100.000 |
| Dibasic Calcium phosphate, anhydrous | 69.750 |
| Microcrystalline cellulose | 69.750 |
| HPMC (e.g. Methocel E5) | 2.750 |
| Carboxymethylcellulose sodium | 5.000 |
| Colloidal silicon dioxide | 1.250 |
| Magnesium stearate | 1.500 |

### Coating

| **Constituents** | **mg/ tablet** |
|---|---|
| HPMC (e.g. Methocel E5) | 2.400 |
| Polyethylene Glycol 6000 | 0.700 |
| Titanium dioxide | 1.043 |
| Talc | 0.857 |
| | |
| **Total Film coated tablet** | **255.000** |

### L) Film coated tablet

### Core

| **Constituents** | **mg/tablet** |
|---|---|
| Flibanserin | 20.000 |
| Lactose monohydrate | 130.000 |
| Microcrystalline cellulose | 43.100 |
| Hydroxypropyl Cellulose (e.g. Klucel LF) | 1.900 |
| Sodium Starch Glycolate | 4.000 |
| Magnesium stearate | 1.000 |

### Coating

| **Constituents** | **mg/ tablet** |
|---|---|
| HPMC (e.g. Methocel E5) | 2.400 |
| Polyethylene Glycol 6000 | 0.700 |
| Titanium dioxide | 1.043 |
| Talc | 0.857 |
| | |
| **Total Film coated tablet** | **205.000** |

## Claims

1. Use of flibanserin, optionally in form the free base, the pharmacologically acceptable acid addition salts and/or optionally in form of the hydrates and/or solvates thereof for the preparation of medicaments useful for the treatment of Insomnia.

2. Use according to claim 1, wherein Insomnia is primary Insomnia.

3. Use according to claim 1, wherein Insomnia is secondary Insomnia.

4. Use according to claim 3, wherein secondary Insomnia is Insomnia secondary to another mental disorder, a general medical condition, or induced by a substance.

5. Use according to claim 3, wherein secondary Insomnia is Insomnia due to a general medical condition.

6. Use according to claim 3, wherein secondary Insomnia is Insomnia induced by a substance.

7. Use according to one or more of claims 1 to 6, **characterized in that** flibanserin is applied in form of flibanserin polymorph A.

8. Use according to one or more of claims 1 to 7, **characterized in that** flibanserin is applied in a dosis range between 0.1 to 400 mg per day.
